Europäisches Patentamt

European Patent Office

Office européen des brevets

(11) Publication number: **0 501 488 A1**

# EUROPEAN PATENT APPLICATION

(21) Application number: **92103383.3**

(22) Date of filing: **27.02.92**

(51) Int. Cl.5: **C07H 1/00**, C07H 13/04, C07H 3/02

(30) Priority: **27.02.91 US 661879**

(43) Date of publication of application:
**02.09.92 Bulletin 92/36**

(84) Designated Contracting States:
**DE FR GB**

(71) Applicant: **MILLIPORE CORPORATION**
**80 Ashby Road**
**Bedford Massachusetts 01730(US)**

(72) Inventor: **Hellerqvist, Carl G.**
**9014 Carondeler Place**
**Brentwood, Tennessee 37027(US)**
Inventor: **Dorschel, Craig A.**
**13 Dodge Avenue**
**Worcester, Massachusetts 01606(US)**

(74) Representative: **Henkel, Feiler, Hänzel &**
**Partner**
**Möhlstrasse 37**
**W-8000 München 80(DE)**

(54) Composition and method for hydrolyzing complex carbohydrates.

(57) Compositions and methods for using them for hydrolyzing complex carbohydrates into their constitutent monosaccharide units are described. The compositions contain a hydrolyzing acid, an acylating agent and an alkylating agent or water. The method involves utilizing the hydrolyzing acid mixture to reduce native and/or derivatized carbohydrates to their individual monosaccharides and simultaneously acylating the clycosidic bonds of the monosaccharides to protect them against further degradation. The acylated monosaccharides are then deacylated to yield the monosaccharide monomers.

Background

Complex carbohydrates are comprised of two or more individual monosaccharides such as, for example, glucose, galactose, mannose, fucose, glucosamine, galactosamine and sialic acid. The latter three sugars may be N-acetylated, and any one may be O-acetylated in one or several positions. These monosaccharides are chemically linked to one another in a particular sequence at specific atomic sites and with a specific stereochemical configuration to form a complex carbohydrate structure.

In order to obtain full knowledge of the structure of an unknown complex carbohydrate, it is necessary to determine the identity and relative concentrations of the constituent monosaccharides, the position(s) in each monosaccharide residue to which another monosaccharide is linked in a glycosidic linkage, the stereochemical configuration (anomeric configuration, $\alpha$ or $\beta$) of each such glycosidic linkage between monosaccharides, and the overall sequence of the monosaccharide units. All of this information is normally required to characterize the structure of a given complex carbohydrate.

In order to obtain this structural knowledge, it is frequently necessary to convert the complex carbohydrate, or chemical derivatives thereof, into the constituent monosaccharides or corresponding chemical derivatives of the same. Acid hydrolysis is commonly used for this purpose. Acid hydrolysis procedures of polysaccharides are well documented. Biermann, In: Hydrolysis and Other Cleavage of Glycosidic Linkages , Biermann et al., (Eds.) "Analysis of Carbohydrates by GLC and Ms", CRC Press, pp. 27-41 (1989). Most commonly used methods employ sulfuric acid, hydrochloric acid or trifluoroacetic acid (TFA). Acid hydrolysis does not break all of the chemical bonds between the constituent monosaccharides at the same rate, rather, individual monosaccharide linkages are hydrolyzed at different rates over the time course required for complete hydrolysis of the complex carbohydrate. As a result, the monosaccharides liberated early in the procedure are subjected to conditions of heat and acidity for a considerably longer period of time than the monosaccharides liberated late in the procedure. Under those conditions, monosaccharides of different types degrade or rearrange (isomerize) at variable rates.

Thus, the stability of individual monosaccharides varies. All monosaccharides in solution present themselves with a "reducing" functional group, either in a "free" form (e.g., aldehyde or ketone) or as a cyclized hemiacetal or ketal form. These forms freely interconvert in solution. Because the reducing group is subject to chemical degradation under conditions of heat and acidity, there is a substantial risk that some of the constituent monosaccharides (or derivatives) obtained from a complex carbohydrate would be lost during the time required for complete hydrolysis of a complex carbohydrate (or derivative), thus leading to erroneous conclusions regarding the structure of the complex carbohydrate. As a result, the conditions (e.g., time, temperature and concentration) which readily release some carbohydrate monomers, such as sialic acid and some deoxy-sugars, are insufficient to effectively release others, such as glucose and mannose.

Harsher conditions are commonly used to liberate amino glycosidic linkages, but those will result in degradation of others. Amino sugars are commonly present in N-acetylated form. This substitution renders the glycosidic linkage labile to acid. A competitive hydrolysis of the N-acetyl group and the glycosidic linkage renders the amino sugar residues which become devoid of the N-acetyl group extremely stable to cleavage of the glycosidic linkage. This problem has been addressed by Hellerqvist et. al., Eur. J. Biochem., 25:96 (1972). Whereas the proposed procedures protect liberated carbohydrate residues, they suffer from several drawbacks which limit their usefulness. For example, lengthy post hydrolysis work-up procedures are needed, and filtration steps reduced the potential recovery of the monomer derivatives. To address today's biologically important problems, a method for hydrolyzing complex carbohydrates which maximizes yield and minimizes degradation of the carbohydrate monomers is needed.

Summary of the Invention

The present invention relates to a method and reagent composition for performing acid hydrolysis of complex carbohydrates. The present composition comprises a mixture of a volatile acid such as a chloro or fluoro derivative of an aliphatic carboxylic acid (e.g., trifluoroacetic acid), an acyl source (i.e., an agent or compound which forms an acyl group), such as acetic acid, and an alkylating agent such as an alcohol, or water. The mixture is used to hydrolyze the carbohydrate bonds. The individual components are added in a sequence determined by the derivatization which the complex carbohydrates have been subjected to prior to the hydrolysis step.

The present method is generally carried out in two steps: hydrolysis and deacylation. The composition of the acid mixture is designed to promote N-acetylation of any free amino groups which may be formed from hydrolysis of an amide group in the complex carbohydrate, as well as to hydrolyze all glycosidic linkages in the first step. This property of the acid mixture will assure a facilitated rapid hydrolysis of the individual N-acetyl-glycosaminidic linkages. Subsequent acylation of the liberated glycosidic hydroxyl groups which occurs reduces the rate of acid-catalyzed degradation of the early released residues which would otherwise occur. In one embodiment, quantitative release of the monosaccharide monomers is achieved by allowing sufficient time to hydrolyze the most stable glycosidic linkages using a proportionately small concentration of trifluoroacetic acid (TFA), acetic acid, water and heat. The reaction conditions are selected to induce quantitative release of the monomers but to minimize degradation of the monomers. When the hydrolysis is complete, the acidic components of the reaction mixture are simply evaporated. In the second step, a mixture of TFA (or other chloro or fluoro aliphatic carboxylic acid) and water is then added, and the mixture is heated in order to hydrolyze all esterified hemi-acetals. After sufficient time, the mixture is evaporated leaving behind a quantitative and qualitative mixture of monomers. This procedure improves on the recovery of the individual monomers over previously used procedures.

The present compositions of acid mixtures and methods of using them provide several advantages. Degradation of carbohydrate monomers is minimized, and excellent quantitative and qualitative monomer recovery is achieved. Post-hydrolysis work-up is simplified which facilitates automation of the procedure. For example, a convenient evaporation method, such as vacuum evaporation, can be used to evaporate the first acid mixture. Addition of the deacylating TFA: water mixture can be automated. Subsequent repeated vacuum evaporation will leave the desired product. The method is rapid and requires no additional vessels, transfer procedures, filtration or solvent removal steps other than evaporation to obtain the carbohydrate monomers.

The present compositions and methods are useful for analytic procedures requiring hydrolysis of complex carbohydrates, or chemical derivatives thereof, and subsequent analysis of the resulting monosaccharide monomers or corresponding derivatives. Degradation of monosaccharides is minimized, which improves the accuracy of subsequent analysis, and allows smaller samples to be used for the entire procedure.

Detailed Description of the Invention

The present reagent compositions and methods provide a process for hydrolyzing carbohydrates. The method allows the sample carbohydrate, native or derivatized, to be hydrolyzed into its corresponding monosaccharide components, which can then can be further derivatized and analyzed, for example, to determine the amount, linkage and anomeric configuration of the carbohydrate. The present compositions and methods can be used to analyze unknown carbohydrates and is particularly useful for hydrolyzing carbohydrates while minimizing degradation of the individual monomers.

The method can be used for analyzing any type of carbohydrate. The term "carbohydrate" as used herein broadly refers to a class of compounds characterized by a carbon backbone, having attached hydrogen and/or hydroxyl groups. Other heteroatoms or groups, e.g., nitrogen or amine groups, can also be attached to the carbon molecules. Carbohydrates include, for example, simple and complex sugars, polysaccharides and oligosaccharides. Generally carbohydrate polymers are made up of at least two individual monosaccharide monomers. The term "monomer" as used herein refers to one individual monosaccharide unit, such as a sugar or sugar derivative (e.g., glucose, galactose, mannose, fucose, glucosamine, galactosamine). "Dimer" refers to two such units, "trimer" to three, etc . . . .

In biological systems, carbohydrates are often bound to macromolecules, such as proteins or lipids, to form glycoconjugates. Glycoproteins, glycolipids and glucosaminoglycans are examples of glycoconjugates. As a consequence of this versatility, the solubility properties of any one of these classes of compounds varies widely both as native compounds and chemical derivatives created for the purpose of performing linkage and anomeric analysis.

The present reagent composition comprises a combination of a chloro or fluoro aliphatic carboxylic acid, an acylating agent (acyl source) and an alkylating agent. A combination of trifluoroacetic acid (TFA), acetic acid and water is the preferred composition. The ratios of TFA, acetic acid and water which are useful for hydrolysis of the carbohydrate bonds without causing significant degradation of the carbohydrate monomers is from about 5:20:75 to about 15:20:65. Other moderately strong to strong volatile acids can be substituted for TFA. Such acids include, for example, chloro and/or fluoro derivatives of aliphatic carboxylic acids (e.g., mono or difluoro acetic acids) and similar analogues having low boiling points either alone or in azeotrope with other solvents, including but not limited to aliphatic alcohols. Hydrochloric acid (HCl) and hydrofluoric acid (HF) can also be used, although organic acids, particularly TFA, are preferred.

Acyl sources which are useful in addition to acetic acid include formic, or propionic acid, or higher alkylcarboxylic acids.

Alkylating agents which can be used in lieu of water include alcohols such as methanol or ethanol. The physical properties of the material to be analyzed determines the ratios of the components to be used and the order of their application to the sample.

The concentration of the acid mixture for the first step depends upon the type, size and chemical nature of the carbohydrates being analyzed. In a preferred embodiment of the method, the concentration of TFA is from about 0.4M to about 1.3M and the concentration of acetic acid is from about 3M to about 12M.

The present method is generally carried out according to the following procedure, which involve two steps: hydrolysis and deacylation. Two general procedures are described, one which is appropriate for native carbohydrates, and the other is most appropriate for derivatived carbohydrates. The term "derivatized carbohydrates" refers to carbohydrates in which one or more of the monosaccharides in the carbohydrate chain is acylated, e.g., having an acetyl or methyl group in one or several positions. To hydrolyze (or depolymerize) native carbohydrates for composition analysis, the carbohydrate of interest is first combined with the water, then contacted with the acids (e.g., TFA and acetic acid) and the combination is heated.

To hydrolyze derivatized (e.g., methylated or acylated) carbohydrates into their corresponding monomers, the carbohydrate is first combined with the acid portions, then the water is added and the mixture is heated. These methods result in the quantitative and qualitative depolymerization of the native or derivatized poly- or oligomeric carbohydrate component by cleavage of the glycosidic bonds joining the individual sugar residues.

The initial hydrolysis step is generally performed at elevated temperatures. Temperatures up to about 120°C are effective for this purpose, without inducing degradation. For most carbohydrates, the hydrolysis step is carried out at a temperature of from about 80°C to about 110°C, preferably about 90°C to about 105°C. The desired time will vary with the temperature. For example, at about 100°C, complete depolymerization generally requires about 2-4 hours, depending on the size of the carbohydrate molecule. Hemiacetals formed in the hydrolysis reaction are protected automatically through acylation, making the total reaction time less critical.

Once the depolymerization hydrolysis is complete, the hydrolyzing acids and water are readily removed from the monomer mixture, e.g., by vacuum evaporation. The resulting acylated monosaccharides produced in the first hydrolysis step are then deacylated in the second deacylation step by hydrolyzing the acyl carboxyl groups. Whereas the depolymerization rate in the first step depends on the individual types of residues and their substitution, making that reaction critical, the deacylation reaction in the second step occurs at very similar rates, independent of the sugars involved. The mechanism of hydrolysis in this step involves the acyl carbonyl group, rather than the glycosidic or ring oxygens of the sugar residues. In the second, deacylation step, the monomer derivatives obtained from the first hydrolysis step, which are partially acylated during the depolymerization reaction, are deacylated into free sugar derivatives by applying to the monomers a combination containing a chloro or fluoro aliphatic carboxylic acid (such as TFA) and water. For this step, the optimum ratio of acid:water is from about 10:90 to about 20:80. The preferred temperature range is about 80°C to 100°C. The reaction is complete within about 30 minutes, which minimizes epimerizations and degradation reactions. The preferred acid component in this step is TFA, but other moderately strong to strong volatile acids can be substituted for TFA. Such acids include other fluoro or chloro derivatives of aliphatic carboxylic acids, HCl and HF.

This two step method minimizes degration of the monomers, and minimizes post-hydrolysis work-up, as no filtration, separation or solvent wash and/or removal steps are necessary. The method is non-invasive and no additional solvents are required. Both steps can be carried out in the same reaction vessel.

Alternatively, the second, deacylating step can be performed by treatment with ammonia in methanol, however, the reaction time is relatively long, which makes this alternative less suitable for automation.

The present reagent compositions and methods can be used to hydrolyze an unknown complex carbohydrate while preserving the monomer units. The reagent composition used in the first step offers several advantages: a quantitative cleavage of all glycosidic bonds; quantitative cleavage of acylated and/or methylated or alkylated oligo- or polysaccharides; continuous N-re-acylation, thereby maintaining the rapid rate of hydrolysis of the corresponding glycosidic residue; protective acylation of hemiacetals formed in the cleavage reaction; depolymerization and acylation for protection of the early released glycosides and the ability to control the order of addition of the reagent components. This maximizes and facilitates solubilization of the native or derivatized carbohydrate, which ultimately determines the quality of the analysis.

The second, deacylation step offers a rapid hydrolysis of the acyl hemiacetal configuration formed in the first step. The rate of hydrolysis is approximately equal for all monomer units due to its relative independence of the involved sugar residue.

In order to obtain complete structural knowledge of an unknown carbohydrate, it is necessary to determine 1) the identity and relative concentrations of the constituent monomers, 2) the sites in each monomer where bonding to another monomer takes place, and 3) the stereochemical configuration (anomeric configuration) of the bonds between the monomers. The common denominator to these three criteria is the first hydrolysis step involved in the hydrolysis of the native, methylated or acetylated carbohydrate component. The combined depolymerization-protection reaction which occurs in the instant method provides the investigator with a quantitative and qualitative sample of the individual components which can be identified after proper derivatization and separation. It is essential in any attempted structural determination to obtain these data.

The present procedure can be automated, thereby expediting and improving the quality of the analysis by minimizing decomposition and loss in the transfer step.

The quantitative hydrolysis and subsequent recovery of the sialyl residues from a glycosyl moiety from feturin was performed using the present protective procedure. The linkage analysis of the lipopolysaccharide with 100 percent recovery of the 3,6-deoxy-hexitol derivative was obtained using the procedure. A combination of TFA, acetic acid and water was used in the first hydrolysis step, and a combination of TFA and water was used in the second deacylating step. When the same carbohydrate was hydrolyzed using the protective procedure described by Hellerqvist et al., only 50% of the acid labile dideoxy sugar was recovered. Hellerqvist et al., Carbohydrate Res., 16:39 (1971).

The composition and/or sequence of a carbohydrate is important for several reasons. Many physical conditions can be detected and/or analyzed by detecting the presence of an oligosaccharide. For example, a test for a pregnancy is based upon the change in N-glycosylation of human serum transcortin and thyroxine binding globulin. Acute inflammation can be determined by measuring the change in hepatic N-glycosylation of $\alpha$-1-antitrypsin and $\alpha$-acid glycoprotein. Knight, Bio-Technology, 7:05-40 (1989) In the biotechnology industry, it is required by regulatory agencies that the structure of the glycosyl moieties resulting from the expression of human proteins in mammalian systems be known for any therapeutic or diagnostic drug.

Equivalents

Those skilled in the art will recognize, or be able to ascertain using no more than routine experimentation, many equivalents to the specific embodiments of the invention described herein. Such equivalents are intended to be encompassed by the following Claims.

## Claims

1. A composition for hydrolyzing and protecting glycosidic bonds in carbohydrates or carbohydrate derivatives, comprising a volatile fluoro or chloro aliphatic carboxylic acid, an acyl source and an alkylating agent or water.

2. The composition of Claim 1 wherein the acid is trifluoroacetic acid.

3. The composition of Claim 1 wherein the acyl source is acetic acid.

4. The composition of Claim 1 wherein the alkylating agent is an alcohol.

5. The composition of Claim 1 comprising trifluoroacetic acid, acetic acid and water.

6. The composition of Claim 5 wherein the ratio of trifluoroacetic acid acid:acetic acid:water is from about 5:20:75 to about 15:20:65.

7. The composition of Claim 2 wherein the concentration of trifluoroacetic acid is from about 0.4M to about 1.3M.

8. The composition of Claim 3 wherein the concentration of acetic acid is from about 3M to about 12M.

9. A method for hydrolyzing a carbohydrate or carbohydrate derivative comprising contacting the carbohydrate with a combination of trifluoroacetic acid, acetic acid and water under conditions sufficient to cause hydrolysis of the carbohydrate bonds to occur.

10. A method of Claim 9 wherein the carbohydrate is a polysaccharide, oligosaccharaide, or an alkylated or acylated derivative thereof.

11. A method of Claim 9 wherein the concentration of trifluoroacetic acid is from about 0.4M to about 1.3M, and the concentration of acetic acid is from about 3M to about 12M.

12. A method of Claim 9 wherein the hydrolysis reaction is performed at a temperature of from about 80°C to about 110°C.

**13.** A method for hydrolyzing a carbohydrate or carbohydrate derivative comprising the steps of:

a. contacting the carbohydrate or derivative with a combination of trifluoroacetic acid and acetic acid at a temperature of up to about 120°C for a time sufficient to cause hydrolysis of the carbohydrate to its substituent monomers to occur;

b. evaporating the trifluoroacetic acid, acetic acid and water from the mixture formed in step (a).

c. contacting the monomers formed in step (b) with a combination of a chloro- or aliphatic carboxylic acid and water at a temperature of up to about 100°C for a time sufficient to de-derivatize the monomers; and

d. evaporating the aqueous acid from the monomer mixture formed after step (c).

**14.** The method of Claim 13 wherein the carbohydrate is a polysaccharide, oligosaccharide, or an alkylated or acylated derivative thereof.

**15.** The method of Claim 13 wherein the evaporation step is performed by vacuum evaporation or by applying a stream of inert gas.

**16.** The method of Claim 13 wherein the concentration of trifluoroacetic acid is about 0.4M to about 1.3M and the concentration of acetic acid is about 3M to about 12M.

**17.** A method of Claim 13 wherein step (a) is performed at a temperature of up to about 100°C.

## DOCUMENTS CONSIDERED TO BE RELEVANT

EP 92103383.3

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 91, no. 5, July 30, 1979, Columbus, Ohio, USA B. LINDBERG et al. "Specific cleavage of O-glycosidic bonds to L-serine and L-threonine by trifluoroacetolysis." page 668, abstract-no. 39 818b  & Acta Chem. Scand., Ser. B. 1979, B 33(3), 230-1  ---- | 1,2,5, 9 | C 07 H   1/00 C 07 H  13/04 C 07 H   3/02 |
| | | | TECHNICAL FIELDS SEARCHED (Int. Cl.5) |
| | | | C 07 H |

The present search report has been drawn up for all claims

| Place of search | Date of completion of the search | Examiner |
|---|---|---|
| VIENNA | 15-05-1992 | SCHNASS |

EPO FORM 1503 03.82 (P0401)